# EUROPEAN PATENT APPLICATION

(11) **EP 2 147 664 A1**
(43) Date of publication of application: **27.01.2010**
(21) Application number: 09166003.5
(22) Date of filing: 21.07.2009
(51) Int. Cl.: A61F 5/01

(54) **Foot and ankle positioning orthosis**

(30) Priority: 22.07.2008 US 177331
(71) Applicant: Orthomerica Products, Inc., Orlando, FL 32810 (US)
(72) Inventor: Varn, Harold T., Braselton, GA 30512 (US); Paaske, Gregory J., Eustis, FL 32736 (US)
(74) Representative: Viering, Jentschura & Partner

(57) **Abstract**

A foot orthosis (2) for un-weighting of a user's heel while promoting a dorsalflexion range of motion includes a leg unit (8) for attachment to a user's leg and a foot unit (10) for supporting a user's foot. A sole plate (6) is configured with a toe extension inclined downward to a first support contact position arch portion extending from the first support contact position to a second support contact pad for support that starts forward of a proximity of a location for a user's calcaneus bone and terminates beneath the calcaneus bone. The foot orthosis enables a gait cycle of movement by the user starting at a heel strike at the second support contact pad and terminating at a toe-off of the toe extension. The sole plate can be attached directly to a support shell (4) to provide a lamination resulting from molding the sole plate to the support shell.

## Description

### BACKGROUND OF THE INVENTION

### 1. Field of the Invention.

The present invention relates to a foot orthosis for promoting a dorsalflexion range of motion, reducing pressure on the toes and the un-weighting of the heel for patients spending prolonged periods of time in a recumbent position.

### 2. Description of Related Art.

Foot orthosis for preventing bedsores on the heels of patients and treating foot drop in patients that are confined to a bed for an extended period of time are known. There is still a need to accommodate patients that can be ambulatory alone or with assistance such as a wheelchair and to provide design features to encourage such ambulatory movement while providing comfort when in a bed.

A foot orthosis utilizing a splint connected to a sole plate has taken various configurations in the prior art such as shown in U.S. Patent Nos. 5,569,173 and 5,735,805.

The orthopedic field, however, is still seeking improvements to provide an economical and effective foot and ankle positioning orthosis.

### SUMMARY OF THE INVENTION

The present invention provides a foot orthosis to assist in preventing heel ulcers on a user with a compromised lower extremity by unloading weight from the heel area to reduce/eliminate pressure on the heel. The foot orthosis further promotes a dorsalflexion range of motion when the use is ambulatory while assisting in prevention of foot drop and ankle contracture.

The foot orthosis can be worn in bed with the user in a supine position and can maintain the lower leg and foot in a neutral position to prevent internal/external rotation of the hip, knee and ankle. A foot covering or bootie can be mounted over the lower portion of the foot orthosis when the user is out of bed.

The foot orthosis includes a leg unit for attachment to a user's leg, and a foot unit for supporting a user's foot relative to the leg unit in a position to un-weight the heel and to protect fragile and compromised skin of the foot and ankle. A calf liner pad of Coolfoam™ and a foot liner pad also of Coolfoam™ is removably mounted for cushioning the user's calf and foot and to maintain a dry contact with the skin. The liner pads are washable and can alternatively constitute one unitary cushioning pad. The liner pads also provide closure straps of a hook and nap material for securing the user's foot to the foot orthosis.

A support shell of an approximately L-shaped configuration can be molded from a plastic material and provides the support structure for the foot orthosis leg unit and foot unit. The lower horizontal portion of the support shell forms a structural support for a sole plate and is configured in a direction from a forward supportive position to receive the user's toes towards a location to receive a heel of the user of a toe extension inclined downward from the forward position to a first support contact position extending traversely across the sole plate in a proximity of a location for a user's sesamoid bone. An intermediate concave arch portion extends from the first support contact position to a second support contact pad for support that starts forward of a proximity of a location for a user's calcaneus bone and terminates beneath the calcaneus bone to enable a normal gait cycle starting at a heel strike at the second support contact paid and terminating at a toe off of the toe extension.

The sole plate can be molded directly to the support shell to provide a lamination with a friction patterned support surface to increase non-slip contact with a support surface. Appropriate holes or apertures can be provided at spaced locations across the support shell to permit an injection molding of the sole plate directly to the support shell.

As a method of manufacturing, the support shall can be mounted in a mold having a cavity of the shape of the sole plate and an injection of a neoprene or a soft polyvinyl chloride can be laminated to form the sole plate for contacting the support surface.

### BRIEF DESCRIPTION OF THE DRAWINGS

The objects and features of the present invention, which are believed to be novel, are set forth with particularity in the appended claims. The present invention, both as to its organization and manner of operation, together with further objects and advantages, may best be understood by reference to the following description, taken in connection with the accompanying drawings.

Figure 1 is a front side perspective view of a foot orthosis and a cover member;

Figure 2 is a rear side perspective view of the foot orthosis with an anti-rotation arm member in dotted lines;

Figure 3 is a front side exploded perspective view of the foot orthosis with separate liner pads;

Figure 4 is a plan view of the support shell with a portion cut out;

Figure 5 is a cross sectional view of a mold with a support shell for laminating the sole plate to the support shell; and

Figure 6 is a partial side view of a laminated support shell and sole plate.

### DETAILED DESCRIPTION OF THE PREFERRED EMBODIMENTS

Reference will now be made in detail to the preferred embodiments of the invention which set forth the best modes contemplated to carry out the invention, examples of which are illustrated in the accompanying drawings. While the invention will be described in conjunction with the preferred embodiments, it will be understood that they are not intended to limit the invention to these embodiments. On the contrary, the invention is intended to cover alternatives, modifications and equivalents, which may be included within the spirit and scope of the invention as defined by the appended claims. Furthermore, in the following detailed description of the present invention, numerous specific details are set forth in order to provide a thorough understanding of the present invention. However, it will be obvious to one of ordinary skill in the art that the present invention may be practiced without these specific details. In other instances, well known methods, procedures, components, molding procedures have not been described in detail as not to unnecessarily obscure aspects of the present invention.

A foot orthosis 2 includes a support shell 4 of an approximately L-shaped configuration to provide support for a sole plate 6 and further to extend up a rear of a heel of a user to terminate at a calf of the user to thereby enable an un-weighting of the heel. The support shell 4 is found of a blend of polymethylmethacrylate and polyvinylchloride to provide a resilient but strong structure. A leg unit 8 is configured on a vertical portion of the support shell 4 complementarily to the shape of the calf of the user with an indented exterior concave portion above the heel while providing clearance space to the rear of the user's heel. The lower or horizontal portion of the support shell 4 is attached to a sole plate 6 and forms a foot unit 10.

A calf liner pad 12 of Coolfoam™, a trademark of Orthomerica Products, Inc. of Orlando, Florida, is attached by a Velcro™ set of hook and tuff material for removal to the support shell 4. Coolfoam™ is a fabric covered pad of a resilient closed cell foam of polyurethane-polyresin. The fabric touching the user can be Orthowick™, a Velcro™ product to whisk moisture away from the user while the exterior fabric of the pads 12 and 14 is a Velcro™ of a nylon loop fabric that is designed to engage a hook material in a fabric format.

A foot liner pad 14 of Coolfoam™ is also attached to the support shell 4 for easy removal to enable a washing of the foot liner pad 14.

Alternatively, a single unitary liner pad (not shown) can replace the calf liner pad 12 and the foot liner pad 14.

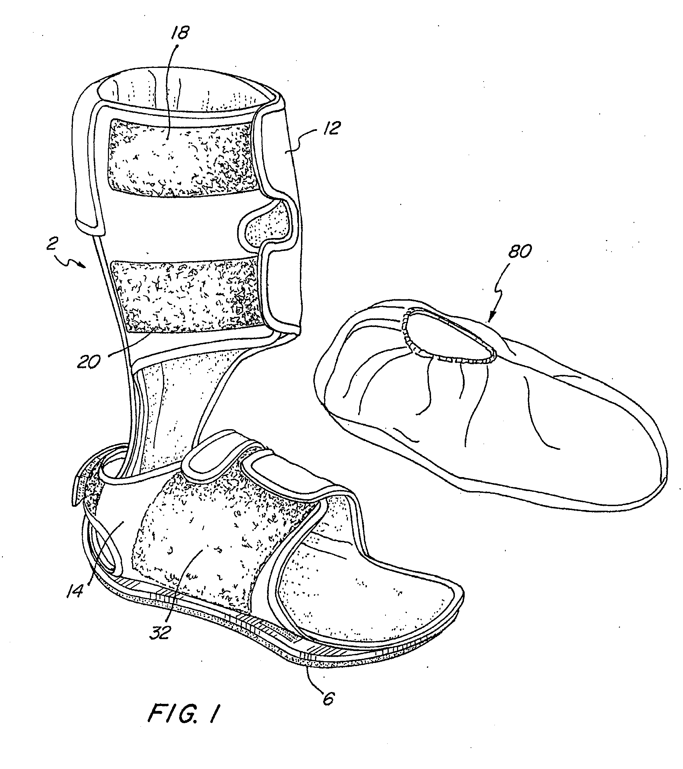

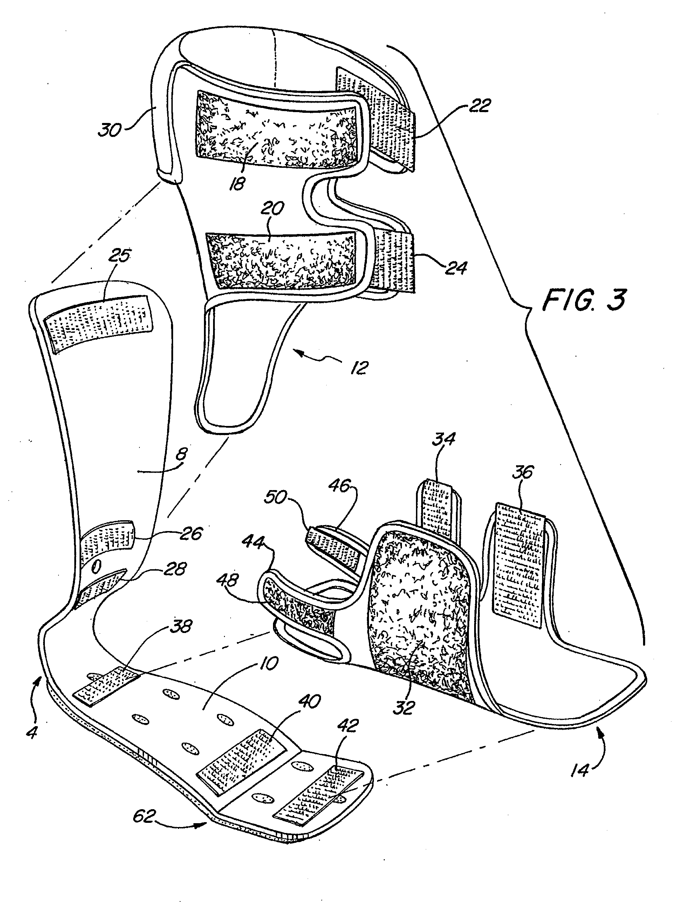

As shown in Figures 1 and 3, the calf liner pad 12 has patches of nap material of a Velcro™ configuration 16 and 18. A complementary hook portion 20 and 22 of Velcro™ permits an adjustable fastening of not only the calf liner pad 12 but also of the leg unit 8 of the support shell 4 to the user.

Strips of hook material 25, 26 and 28 are mounted by adhesives on the leg unit 8 for directly securing to a nap-like exterior fabric surface of the calf liner pad 12. A folded lip section 30 on the back of the calf liner pad 12 folds over the top of the leg unit 8 to capture the leg unit 8 and provides a soft edge to the user. The folded lip section can be sewn to provide a pocket for capturing the top of the support shell 4.

The foot liner pad 14 includes a swath of nap material 32 to provide an anchor position for extended straps 34 and 36, each of which supports a band of hook material. This arrangement permits an adjustable securement of the foot unit 10 to the patient. The horizontal section of the support shelf 4 forms the foot unit 10 and likewise has strips of hook material 38, 40 and 42 held by adhesives on the upper surface for securement of the foot liner pad 14 to the foot unit 10.

A pair of strap sections 44 and 46, at the rear of the foot liner pad 14, extend an appropriate length to permit encircling and capturing the rear surface of the leg unit 8 across the indented portion of the leg unit 8 of the support shell 4. Strap 44 has an exterior strip of nap material 48 while strap 46 has an interior strip of hook material complementary to the nap material 48 to permit an adjustable mounting of the foot liner pad 14 so that it is secured along the horizontal foot unit 8 by the interface with the respective strips of hook material 38, 40 and 42 and an adjustable securement of the strap sections 44 and 46.

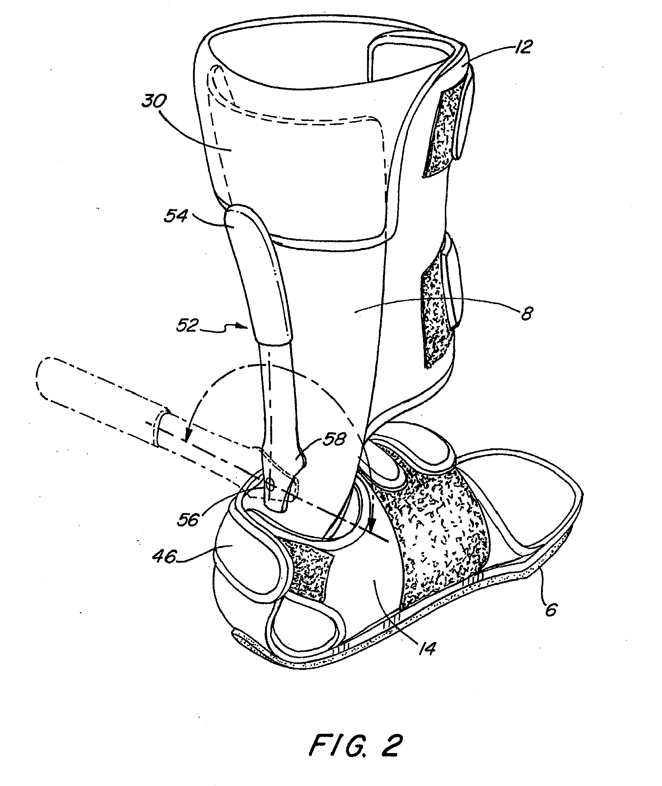

Referring to Figure 2, an anti-rotation pivotally mounted arm 52 is disclosed in vertical alignment with the rear of the leg unit 8 which is the position utilized for walking. A plastic sleeve 54 can be slid over the end of the arm 52. The anti-rotation arm 52 can be formed of a blend of polymethylmethacrylate and polyvinylchloride in the same manner as the support shell 4.

A rivet 56 or other fastener that is connected to the support shell 4 permits a rotational movement of the anti-rotation arm 54. A symmetrical camming extension 58 on either side of a lower portion of the anti-rotational arm 52 adjacent the pivot assists in holding an offsetting of the anti-rotation arm 52 when it rotates from a vertical position to a horizontal position with a curved surface matching the exterior contour of the leg unit 8, thereby assisting in maintaining the anti-rotational arm 52 in a horizontal position at 90° to either the right or left side of Figure 2. The anti-rotational arm 52 further assists in limiting movement of the foot orthosis when the patient is lying in a bed. Basically, the curved heel portion of the foot orthosis 2 and the laterally extended anti-rotational arm 52 at the top edge of the leg unit 8 will contact the bed surface and maintain the user's foot extending vertically upward. As can be further appreciated, the foot unit 10 is dimensioned in length to prevent the weight of any bed covers from directly contacting the user's toes.

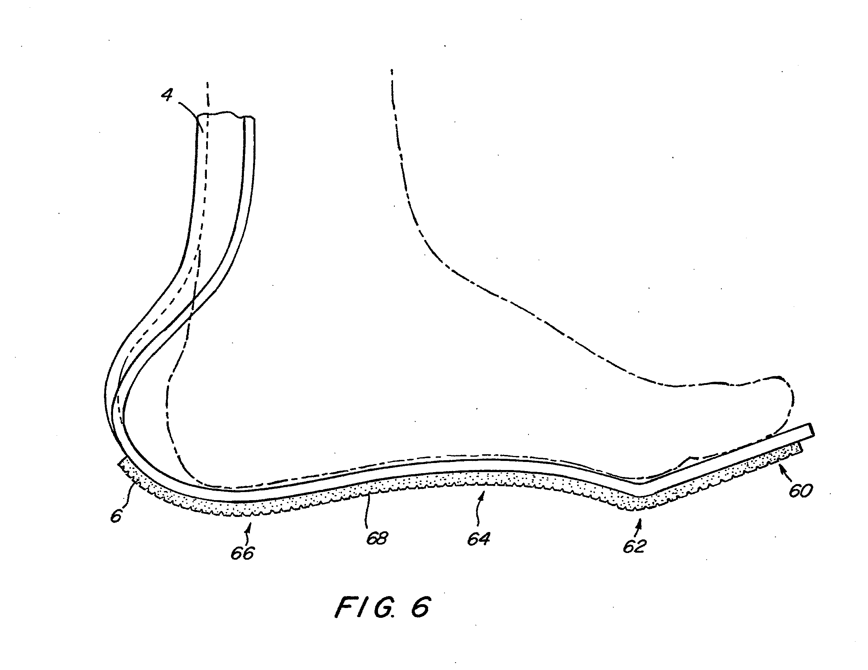

Referring to Figure 6, a side partial cross-sectional view of the support shelf 4 and the sole plate 6 is shown. The horizontal configuration of the support shelf 4 which forms the basis for the foot unit 10 also defines the configuration of the sole plate 6.

The sole plate 6 is configured in a direction from a forward supported position to receive the toes of the user towards a location to receive the heel of a user. A toe extension 60 is inclined downward in a planar configuration to a first support contact position 62 which extends linearly and transversely across the sole plate 6 in a proximity of a location to receive a user's sesamoid bone. An intermediate concave arch portion 64 extends rearward from the first support contact position 62. A second support contact position 66 of a convex configuration is configured for support that will start somewhat forward of a proximity of a location to receive a user's calcaneus bone and will terminate beneath the calcaneus bone. As a result, the heel of the user is un-weighted to help prevent pressure ulcers.

In a walking mode, a normal gait cycle of user movement starting at a heel strike of the second support contact position 66 and terminating at a toe-off of the toe extension 60 may be employed by the user. The sole plate 6 has an irregular pattern surface 68 to increase a friction contact with the support surface during walking.

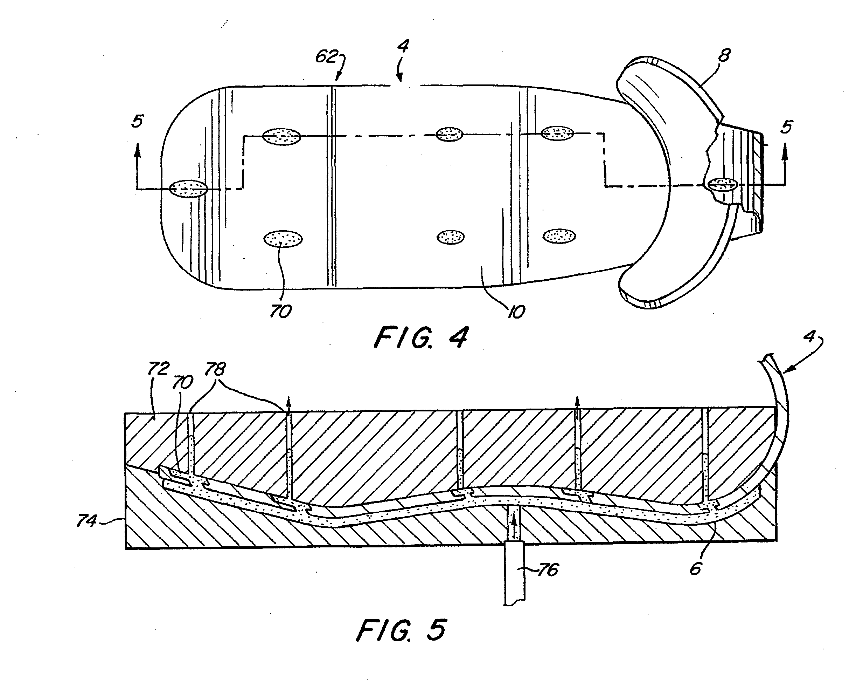

Additionally, the sole plate 6 can be laminated directly to the support shelf 4. Referring to Figures 4 and 5, a plan view of the support shelf 4, having surface apertures of an enlarged entrance 70 are located across the upper surface of the support shelf 4 on the foot unit 10. These enlarged areas receive an anchor portion with a neck down connection to the sole plate 6.

Referring to Figure 5, an upper mold half 72 is indented to receive and complement the foot unit 10 of the support shelf 4 which can be cast to have the appropriate configurations for the surface apertures 70. A lower mold half 74 is configured to support and also receive support shelf 4 and to create a cavity configured to enable the sole plate to be injection molded and laminated within the closed molded set with the support shelf 4.

The injection port 76 can inject the appropriate neoprene or soft polyvinylchloride material into the mold and a series of vent ports 78 can ensure that the injected material fills the cavity configuration of the sole plate 6 and vents air. Subsequently, the vent port runners are removed in a post molding procedure and the permanently attached sole plate 6 is laminated directly to the bottom of the support shell 4, forming the foot unit 10.

When the patient wishes to leave the bed, they can don the bootie 80 that covers the foot unit 10 with an elastic opening that can be stretched to accommodate the foot orthosis 2. The bootie can have a friction dimpled under surface to prevent slipping.

The foot orthosis 2 can be fitted to a patient in a supine position, the knee flexed while flexing the toes of the patient in order to position the heel down and into contact with the leg unit 8 for correctly positioning the heel. As can be seen in the phantom lines of Figure 6, the heel of the patient is in contact with the plantar surface of the orthosis and correctly seated with a space at the vertical back of the heel. Thus, the heel is un-weighted to help prevent pressure ulcers. Additionally, this unloading assists in the treatment of a pre-existing ulcer on a posterior aspect of the user's calcaneus.

With the anti-rotation arm 52 of the present invention, the desired post-surgical positioning of the lower limb can be facilitated by preventing the lower limb internal or external rotation when the patient is in a supine position in the bed.

Additionally, the foot orthosis 2 assists in the protection of fragile and compromised skin of the foot and ankle can address foot drop with contractures. In picking the size of the foot orthosis 2, toes should be recessed from the forward position of the toe extension 60, thereby facilitating the supportive weight of any covers or bedding material when the patient is in a supine position. The angle of the toe lift extension 60 also facilitates clearance in a swinging movement during ambulation of the user.

The foot orthosis 2 can also have a universal fit in that it can be utilized on either the right or left foot of the patient.

The use of the Velcro™ tabs facilitate an independent donning and doffing of the foot orthosis 2 by the patient. The calf liner pad 12 has a proximal wrap around the trim line of the upper edge of the leg unit 8 which overlaps and captures the upper edge of the support shelf 4 to reduce any calf pressure.

The relative flexibility of the support shell 4 material improves clearance in the swing motion, further promoting a neutral position in stance phase for limited ambulation.

By utilizing a laminated walker bottom to the foot unit 10, there is no necessity in utilizing additional tools. The external cover or bootie 80 with a resilient elastic opening and dimpled portion bottom surface helps prevent pathogen transfer when worn over the foot orthosis 2, but realistically is not always utilized by the patient. The walker bottom, however, has a friction surface and its configuration of a first support contact position 62, an intermediate concave arch portion 64 and a second support contact position 66 promotes a dorsalflexion range of motion and enables a relatively normal gait cycle of user movement.

Those skilled in the art will appreciate that various adaptations and modifications of the just-described preferred embodiment can be configured without departing from the scope and spirit of the invention. Therefore, it is to be understood that, within the scope of the amended claims, the invention may be practiced other than as specifically described herein.

## Claims

1. A foot orthosis for un-weighting of a user's heel while promoting a dorsalflexion range of motion comprising:
a leg unit for attachment to a user's leg; and
a foot unit for supporting a user's foot relative to the leg unit including a sole plate configured, in a direction from a forward supportive position to secure toes towards a location to receive a heel of the user, of a toe extension inclined downward from the forward supportive position to a first support contact position extending traversely across the sole plate in a proximity of a location to receive the user's sesamoid bone, an intermediate concave arch portion extending from the first support contract position to a second support contact pad for support that starts forward of a proximity of a location to receive a user's calcaneus bone and terminates beneath the calcaneus bone to enable a normal gait cycle of user movement starting at a heel strike at the second support contact pad and terminating at a toe off of the toe extension.

2. The foot orthosis of Claim 1 further including an approximately L-shaped support shell configured to support the sole plate and further to extend upward where a rear of a heel would be located while enabling an un-weighing of the heel of the user.

3. The foot orthosis of Claim 2 wherein the sole plate is attached directly to the support shell to provide an integral lamination resulting from molding the sole plate to the support shell.

4. The foot orthosis of Claim 2 wherein the L-shaped support shell includes a plurality of spaced apertures extending through the support shell and the sole plate includes a plurality of complimentary posts, each extending through respective spaced apertures with anchor heads configured on the posts for retaining the sole plate to conform to the shape of the foot unit.

5. The foot orthosis of Claim 2 wherein the support shell further extends upward from the rear of the heel and is configured as a leg portion to conform to a back of a user's lower leg.

6. The foot orthosis of Claim 2 wherein an anti-rotation arm member is pivotally mounted on the support shell and configured to provide lateral support to limited movement of the foot orthosis when the user is in a supine position.

7. The foot orthosis of Claim 6 wherein the anti-rotation arm member has a pivot point connector with a pair of side camming faces to provide a 90° location on either side of a pivot point for the anti-rotational arm member from a vertical alignment with the support shell at a fixed storage location for walking.

8. The foot orthosis of Claim 2 further including a foot liner pad removably attached to the support shell.

9. The foot orthosis of Claim 8 further including a pair of heel straps on the foot liner pad for removably extending around an indented portion of the upwards portion of the support shell.

10. The foot orthosis of Claim 9 wherein an anti-rotational arm member is pivotally mounted on the support shell, adjacent the indented portion, and configured to provide lateral support to limited movement of the foot orthosis when the user is in a supine position.

11. The foot orthosis of Claim 10. wherein the anti-rotational arm member includes a symmetrical camming extension adjacent the pivotal mounting to assist in holding the anti-rotational arm member when extended at 90° from an alignment with the leg unit.

12. The foot orthosis of Claim 2 further including a calf liner pad removably attached to the support shell, the calf liner pad having an extended lip configured to be bent over an upper edge of the L-shaped support shell.

13. The foot orthosis of Claim 2 further including an external cover bootie with an elastic opening configured to receive the user's foot and the foot unit.

14. A foot orthosis for un-weighting of a user's heel while promoting a dorsalflexion range of motion comprising:
a leg unit for attachment to a user's leg;
a foot unit for supporting a user's foot relative to the leg unit including a sole plate configured, in a direction from a forward supportive position to secure toes towards a location to receive a heel of the user, of a toe extension inclined downward from the forward supportive position to a first support contact position extending traversely across the sole plate in a proximity of a location to receive the user's sesamoid bone, an intermediate concave arch portion extending from the first support contract position to a second support contact pad for support that starts forward of a proximity of a location to receive a user's calcaneus bone and terminates beneath the calcaneus bone to enable a normal gait cycle of user movement starting at a heel strike at the second support contact pad and terminating at a toe off of the toe extension;
a unitary plastic support shell is configured to support the sole plate and extend upward as support for the leg unit;
a foot liner pad is removably fastened directly to the support shell with straps to hold the user's foot; and
a calf liner pad is removably fastened directly to the support shell with straps to hold the user's lower leg.

15. A foot orthosis for un-weighting of a user's heel while promoting a dorsalflexion range of motion comprising:
a leg unit for attachment to a user's leg;
a foot unit for supporting a user's foot relative to the leg unit including a sole plate configured, in a direction from a forward supportive position to secure toes towards a location to receive a heel of the user, of a toe extension inclined downward from the forward supportive position to a first support contact position extending traversely across the sole plate in a proximity of a location to receive the user's sesamoid bone, an intermediate concave arch portion extending from the first support contract position to a second support contact pad for support that starts forward of a proximity of a location to receive a user's calcaneus bone and terminates beneath the calcaneus bone to enable a normal gait cycle of user movement starting at a heel strike at the second support contact pad and terminating at a toe off of the toe extension;
an approximately L-shaped support shell configured to support the sole plate and further to extend up where a rear of a heel would be located while enabling an un-weighting of the heel of the user, wherein the sole plate is attached directly to the support shell to provide a lamination resulting from molding the sole plate to the support shell; and
an anti-rotational arm member is pivotally mounted on the support shell and includes a symmetrical camming extension adjacent the pivotal mounting to assist in holding the anti-rotational arm member when extended at 90° from an alignment with the leg unit.
